# EUROPÄISCHE PATENTSCHRIFT

(11) **EP 1 658 815 B1**
(45) Veröffentlichungstag und Bekanntmachung des Hinweises auf die Patenterteilung: **28.03.2012**
(21) Anmeldenummer: 05023531.6
(22) Anmeldetag: 27.10.2005
(51) Int. Cl.: A61B 17/70

(54) **Elastisches Element zur Verwendung in einer Stabilisierungseinrichtung für Knochen oder Wirbel**
Elastic element for use in a stabilising device for bones or vertebrae
Elément élastique pour système de stabilisation d'os ou de vertèbres

(30) Priorität: 17.11.2004 DE 102004055454; 17.11.2004 US 628811 P
(43) Veröffentlichungstag der Anmeldung: 24.05.2006
(73) Patentinhaber: BIEDERMANN MOTECH GmbH, 78054 Villingen-Schwenningen (DE)
(72) Erfinder: Biedermann, Lutz, 78048 VS-Villingen (DE); Matthis, Wilfried, 79367 Weisweil (DE); Harms, Jürgen Prof. Dr., 76227 Karlsruhe (DE)
(74) Vertreter: Hofer, Dorothea

(56) Entgegenhaltungen:
- EP-A- 0 667 127
- EP-A- 0 677 277
- WO-A-2005/084567
- FR-A- 2 799 949
- SU-A1- 1 102 585
- US-A- 5 984 925
- US-A1- 2004 073 215

## Beschreibung

Die vorliegende Erfindung betrifft ein elastisches Element für die Verwendung in der Wirbelsäulen- oder Unfallchirurgie, ein Knochenverankerungselement, ein stabförmiges Element und eine Stabilisierungseinrichtung jeweils mit einem solchen elastischen Element.

Zur Fixierung von Knochenfrakturen oder zur Stabilisierung der Wirbelsäule sind Fixations- und Stabilisierungseinrichtungen bekannt, die aus wenigstens zwei im Knochen bzw. Wirbel verankerten und über eine Platte oder über einem Stab verbundenen Knochenschrauben bestehen. Derartige starre Systeme erlauben keine Bewegung der relativ zueinander fixierten Knochenteile oder Wirbel.

Für bestimmte Indikationen ist jedoch eine dynamische Stabilisierung wünschenswert, bei der die zu stabilisierenden Knochenteile und Wirbel eine kontrollierte begrenzte Bewegung zu einander ausführen können. Eine Möglichkeit für die Realisierung der dynamischen Stabilisierungseinrichtung besteht in der Verwendung eines elastischen Elementes anstelle eines die Knochenverankerungselemente verbindenden starren Stabes.

Aus der US 2003/0 191 470 A1 (Basis für den Oberbegriff des Anspruchs 1) ist ein elastisches Element zur Verbindung von Knochenschrauben bekannt, das bei Auslenkung aus einer Ruhelage eine rücktreibende Kraft ausübt. Das elastische Element weist durch eine einseitig der Stabachse angeordnete schleifenartige Ausbildung des Stabmittelbereichs eine asymmetrische Form auf und es treten lokale starke Belastungen des Stabes auf.

In der US 2004/0073215 A1 ist eine dynamische Zwischenwirbelverbindungsvorrichtung offenbart, deren Enden zum Befestigen an Wirbeln ösenartig ausgebildet sind.

Die FR 2 799 949 A1 offenbart ein Element zum Verbinden von Wirbelkörpern mit einem Abschnitt in Form einer schraubenförmigen Feder.

Die EP 677 277 A2 offenbart ein Element zum Verbinden von Wirbeln mit einem im Wesentlichen zylinderförmigen elastischen Abschnitt.

Aus der US 6,440,169 B1 ist ein elastisches Element zur Stabilisierung von Wirbeln bekannt, bei dem zwei als Blattfedern ausgebildete Elemente eine begrenzte Kompressionsbewegung entlang der Verbindungsachse der Wirbel ermöglichen.

Aus der US 2003/0 220 643 A1 ist ein Stab zur Verbindung von zwei Knochenschrauben bekannt, der einen schraubenfederförmigen elastischen Abschnitt aufweist. Die Biegesteifigkeit dieses elastischen Abschnitts ist in allen Richtungen senkrecht zur Stabachse gleich und somit liegt keine gerichtete Biegesteifigkeit vor.

Es ist Aufgabe der Erfindung, ein verbessertes elastisches Element bereit zu stellen, das senkrecht zu der Stabachse eine richtungsabhängige Biegesteifigkeit und eine hohe Festigkeit bei zyklischer Belastung aufweist und das mit anderen Elementen in möglichst vielfältiger Weise zu einer dynamischen Stabilisierungseinrichtung für Wirbel oder Knochen kombiniert werden kann.

Die Aufgabe wird gelöst durch ein elastisches Element nach dem Patentanspruch 1.

Weiterbildungen der Erfindung sind in den Unteransprüchen angegeben.

Die Erfindung weist den Vorteil auf, dass das elastische Element zugleich kompakt und mit einer richtungsabhängigen Biegesteifigkeit ausgebildet ist. Dies ist insbesondere für Anwendungen an der Wirbelsäule und dort insbesondere an der Halswirbelsäule von Bedeutung, wo der zu Verfügung stehende Platz im Vergleich zu Anwendungen an der Lendenwirbelsäule deutlich geringer ist. Ferner kann die Geometrie leicht an unterschiedliche Anforderungen angepasst werden, da eine hohe Bandbreite des Federverhaltens erreicht werden kann.

Die Erfindung weist weiterhin den Vorteil auf, dass aufgrund einer annähernd symmetrischen Ausbildung bezüglich der Verbindungsachse der beiden Enden die rücktreibende Kraft symmetrisch bezüglich entgegengesetzter Auslenkung aus der Ruhelage ist und die Materialbelastung bei zyklischer Belastung gleichmäßiger verteilt ist, als bei den bekannten elastischen Elementen, was die Lebensdauer erhöht und die Gefahr von Brüchen aufgrund von Materialermüdung reduziert.

Weiterhin ist eine über die mittlere Länge nahezu konstante Biegespannung erreicht. Durch die dynamische axiale Einfederung ergibt sich ein Vorteil der Erhaltung der translatorischen Bewegung bei der Verwendung an einem Facettengelenk, wodurch Arthrose vermieden wird.

Ferner weist die Erfindung den Vorteil auf, dass ein elastisches Element nach Wahl mit anderen Elementen zur dynamischen Stabilisierung kombiniert werden kann, sodass ein hohes Maß an Anpassbarkeit an individuelle Bedürfnisse gegeben ist.

Weitere Merkmale und Zweckmäßigkeiten ergeben sich aus der Beschreibung von Ausführungsbeispielen anhand der Figuren.

Von den Figuren zeigen:

Weitere Merkmale und Zweckmäßigkeiten der Erfindung ergeben sich aus der Beschreibung von Ausführungsbeispielen anhand der beigefügten Zeichnungen. Von den Figuren zeigen:
- Fig. 1: eine perspektivische Ansicht eines elastischen Elements nach einer ersten Ausführungsform;
- Fig. 2: eine Seitenansicht des elastischen Elements nach der ersten Ausführungsform;
- Fig. 3: eine perspektivische Detaildarstellung eines Abschnitts des elastischen Elements nach der ersten Ausführungsform;
- Fig. 4: eine perspektivische Ansicht eines elastischen Elements nach einer zweiten Ausführungsform;
- Fig. 5: eine Seitenansicht des elastischen Elements nach der zweiten Ausführungsform;
- Fig. 6: eine perspektivische Ansicht eines elastischen Elements nach einer dritten Ausführungsform;
- Fig. 7: eine Seitenansicht des elastischen Elements nach der dritten Ausführungsform;
- Fig. 8: eine perspektivische Ansicht eines elastischen Elements nach einer vierten Ausführungsform; und
- Fig. 9: eine teilgeschnittene schematische Darstellung einer Anwendung einer Stabilisierungseinrichtung mit einem elastischen Element.

### Erste Ausführungsform

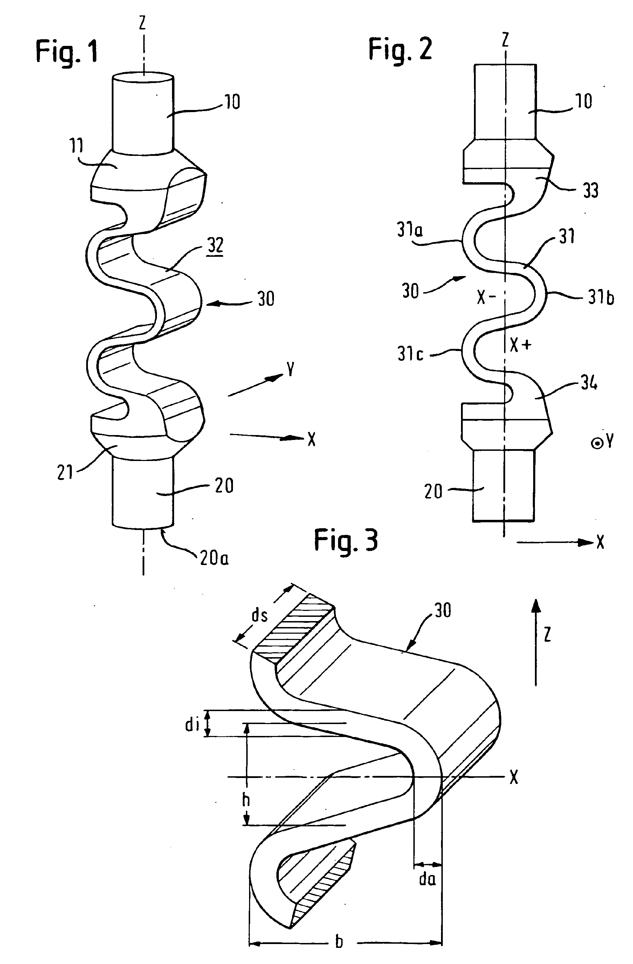

Im Folgenden wird mit Bezug auf Fig. 1 und 2 eine erste Ausführungsform der vorliegenden Erfindung beschrieben.

Bei der in den Fig. 1 und 2 gezeigten ersten Ausführungsform weist das elastische Element ein erstes Ende 10, ein zweites Ende 20 und einen dazwischen angeordneten elastischen Abschnitt 30 auf, die einstückig ausgebildet sind. Das elastische Element ist aus einem körperverträglichen Material wie zum Beispiel Titan gefertigt, es kann auch aus einer körperverträglichen Formgedächtnislegierung, die Superelastizität aufweist, wie z.B. Nitinol, gefertigt sein.

Das erste Ende 10 und das zweite Ende 20 sind mit zylindrischem Querschnitt ausgebildet, wobei die Zylinderachsen parallel zu der Verbindungsachse Z des ersten Endes 10, des elastischen Abschnitts 30 und des zweiten Endes 20 angeordnet sind. In Richtung des Abschnitts 30 schließt sich an das erste Ende 10 ein konischer Abschnitt 11 an, der sich von dem Zylinderquerschnitt des ersten Endes 10 auf den Querschnitt des elastischen Abschnitts 30 konisch verbreitert. Gleichermaßen schließt sich das zweite Ende 20 ein konischer Abschnitt 21 an.

Der elastische Abschnitt 30 schließt sich an den konischen Abschnitt 11 an und ist in Form eines schleifenförmig geformten Flachstabes 32 mit im Wesentlichen rechteckigem Querschnitt ausgebildet. Wie in Fig. 2 in einer Seitenansicht dargestellt ist, verläuft die Schleifenform des elastischen Abschnitts 30 von dem konischen Abschnitt 11 zu dem konischen Abschnitt 12 und Schleifenbäuche 31 erstrecken sich im Wesentlichen in einem sinusartigen Verlauf in einer Richtung X senkrecht zur Verbindungsachse Z abwechselnd zu der einen Seite X+ und zu der anderen Seite X- der Verbindungsachse. In den Fig. 1 und 2 sind beispielhaft auf der Seite X- zwei Schleifenbäuche 31a, 31c und auf der X+ Seite ein Schleifenbauch 31b dargestellt. Die Anzahl der Schleifenbäuche kann jedoch nach den gewünschten Eigenschaften des elastischen Elements gewählt werden. Ferner können die Länge der zylindrischen Enden 10, 20 und die Länge des elastischen Abschnitts 30 bei einer Verwendung in einer Stabilisierungseinrichtung für Knochen und Wirbel entsprechend dem Abstand der Verankerungselemente und den erforderlichen elastischen Eigenschaften des elastischen Elements gewählt werden.

In einer Richtung Y, die senkrecht zu der Verbindungsachse Z und der Richtung X angeordnet ist, weist der elastische Abschnitt 30 über seine gesamte Länge eine konstante Breite ds auf. Der schleifenförmig augebildete Flachstab 32 weist entlang seines Verlaufs senkrecht zu der Richtung Y in der gezeigten Ausführungsform außer in den unmittelbar an den verbreiterten Rand 11 und den verbreiterten Rand 12 anschließenden Abschnitten 33 und 34, in denen ein größerer Durchmesser gegeben ist, einen konstanten Durchmesser auf.

Im Folgenden wird der elastische Abschnitt 30 anhand von Fig. 3 eingehender beschrieben. In Fig. 3 sind die Abmessungen des elastischen Abschnitts, die einen wesentlichen Einfluss auf die elastischen Eigenschaften haben, im Detail dargestellt. Durch Variieren der Parameter ds (Breite des elastischen Abschnitts in der Richtung Y), b (doppelte Amplitude der Welle), h (halbe Wellenlänge), da (Materialdicke am Schleifenbauch in Richtung X) und di (Materialdicke im Nulldurchgang (Verbindungsachsendurchgang) in Richtung der Verbindungsachse Z) können die elastischen Eigenschaften des elastischen Abschnitts an die gewünschten Erfordernisse angepasst werden. Diese Einflüsse werden im Folgenden mit Bezug auf Fig. 3 diskutiert.

Durch die Ausführung des elastischen Abschnitts 30 in Form eines schleifenförmig geformten Flachstabes 32 wird erreicht, dass das elastische Element eine hohe Torsionssteifigkeit bezüglich Torsion um die Verbindungsachse Z und gleichzeitig eine große Biegesteifigkeit bezüglich Biegebelastung in der Richtung Y, d.h. eine Biegung um eine in Richtung X verlaufende Achse, aufweist. Hingegen ist eine hohe Elastizität bezüglich einer Biegebelastung in Richtung X, d.h. einer Biegung um eine in Richtung Y verlaufende Achse, und falls gewünscht auch eine hohe Elastizität bezüglich Kompression und Extension in der Richtung der Verbindungsachse Z ermöglicht.

Z.B. können durch eine Vergrößerung der Breite ds des elastischen Abschnitts in der Richtung Y gleichzeitig die Torsionssteifigkeit und die Biegesteifigkeit in der Richtung Y erhöht werden. Durch entsprechende Wahl der anderen Parameter h, da, di und b können die Biegesteifigkeit und das elastische Federungsverhalten entlang der Verbindungsachse Z gezielt eingestellt werden.

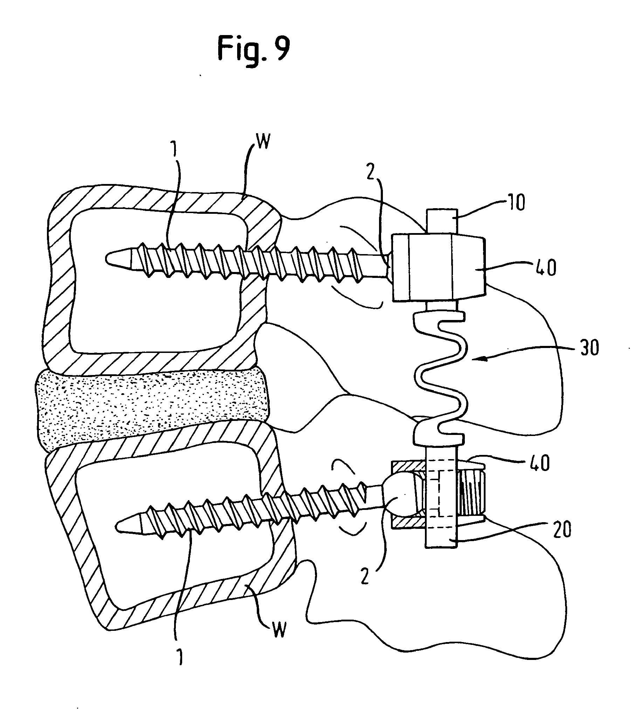

In Fig. 9 ist eine beispielhafte Verwendung des elastischen Elements schematisch dargestellt, bei der das erste und das zweite Ende 10, 20 jeweils in einem Aufnahmeteil 40 einer Polyaxialknochenschraube aufgenommen sind. Die Polyaxialknochenschrauben sind dabei mit ihren Schäften 1 in benachbarten Wirbeln W einer Wirbelsäule verankert und die Köpfe 2 der Knochenschrauben sind in dem jeweiligen Aufnahmeteil 40 schwenkbar und über ein Fixierelement in ihrer Winkelstellung arretierbar gehalten.

In einer solchen Anordnung wird durch die Verwendung des elastischen Elements eine kontrollierte Bewegung zwischen den Wirbeln dadurch ermöglicht, dass eine elastische Translationsbewegung in Richtung der Verbindungsachse Z des elastischen Elements und eine elastische Biegebewegung in Richtung X ermöglicht sind, wohingegen eine Torsionsbewegung und eine Biegebewegung in Richtung Y weitgehend unterbunden sind.

Durch geeignete Wahl der mit Bezug auf Fig. 3 beschriebenen Parameter können die gewünschten Eigenschaften des elastischen Elements bezüglich der kontrollierten Bewegung auf einfache Weise eingestellt werden und mit elastischen Elementen unterschiedlicher Eigenschaften in Kombination mit Monoaxial- oder Polyaxialknochenschrauben und Stäben oder Platten ist eine hohe Variabilität gegeben.

### Zweite Ausführungsform

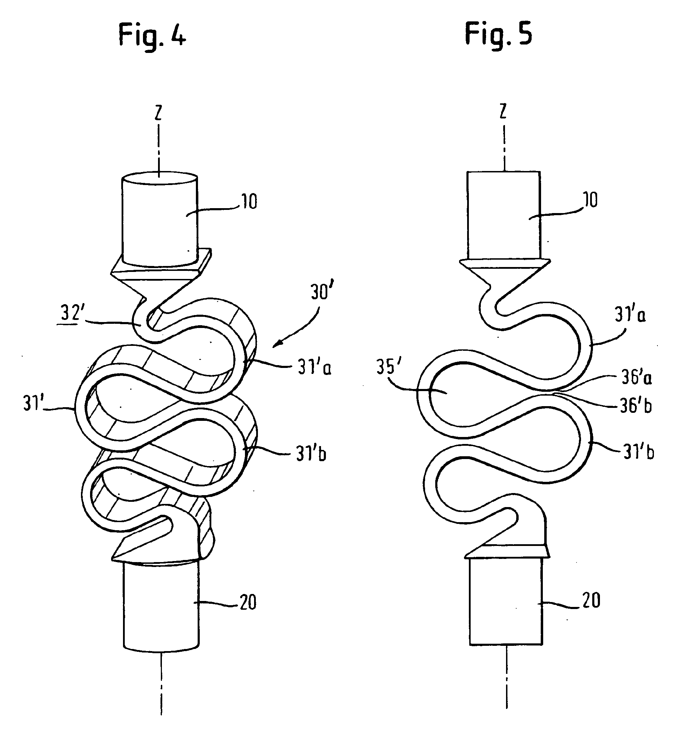

Die in den Fig. 4 und 5 dargestellte zweite Ausführungsform unterscheidet sich von der ersten Ausführungsform im Wesentlichen nur in der Ausbildung des elastischen Abschnitts. Bei der zweiten Ausführungsform ist der elastische Abschnitt 30' wie bei der ersten Ausführungsform in Form eines schleifenförmig geformten Flachstabes 32' ausgebildet. Die Schleifenform unterscheidet sich von der bei der ersten Ausführungsform darin, dass nicht ein sinusartiger sondern ein schleifenförmiger bzw. mäanderartiger Verlauf gegeben ist, bei dem die Schleifenbäuche 31' eine bauchigere Form aufweisen, die in der Seitenansicht tropfenförmige freie Bereiche 35' einschließen. Bei dieser Ausführungsform weisen die Seitenflächen 36' zweier benachbarter Schleifenbäuche 31'a und 31'b verglichen mit der ersten Ausführungsform nur einen geringen Abstand zueinander auf.

Durch diese spezielle Ausführung kann ein elastisches Einfedern entlang der Verbindungsachse Z beschränkt werden und dabei gleichzeitig durch geeignete Wahl der anderen Parameter, wie bei der ersten Ausführungsform, die Biegesteifigkeit bezüglich der anderen Richtungen je nach Bedarf eingestellt werden.

### Dritte Ausführungsform

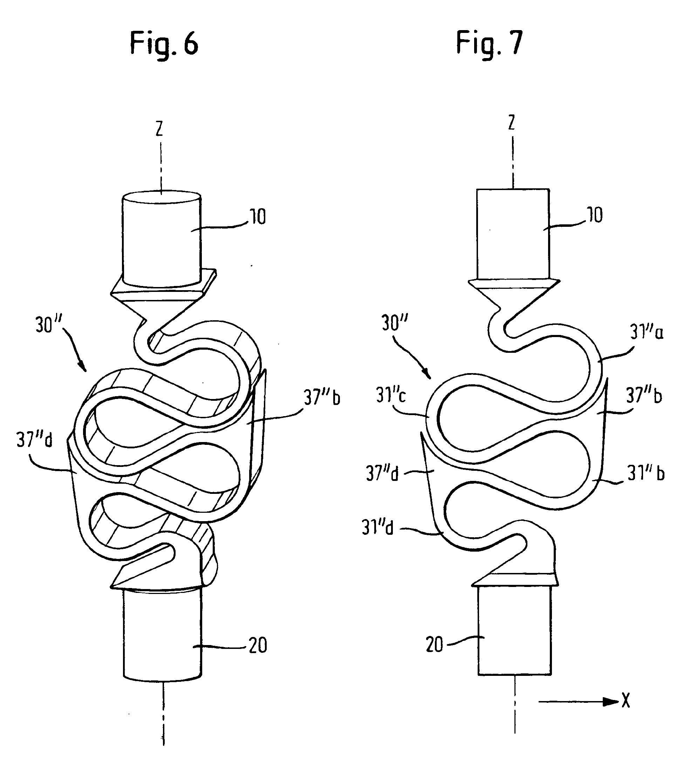

Die in den Fig. 6 und 7 dargestellte dritte Ausführungsform unterscheidet sich von der zweiten Ausführungsform darin, dass der elastische Abschnitt 30" zwischen benachbarten Schleifenbäuchen 31''a und 31''b einen mit dem Schleifenbauch 31"b einstückig verbundenen Fortsatz 37"b sowie auf der gegenüberliegenden Seite der Verbindungsachse Z zwischen den benachbarten Schleifenbäuchen 31"c und 31"d einen mit dem Schleifenbauch 31"d einstückig verbundenen Fortsatz 37''d aufweist.

Die Fortsätze 37"b und 37''d sind dabei derart ausgebildet, dass ihre dem benachbarten Schleifenbauch 31''a bzw. 31"c zugewandte Seitenfläche im Wesentlichen dessen Verlauf folgt und einen geringen Abstand zu diesem aufweist. Eine weitere Außenfläche verläuft im Wesentlichen entlang der Verbindungslinie von dem Schleifenbauch 31"b bzw. 31"d zu dem benachbarten Schleifenbauch 31"a bzw. 31"c, jedoch ohne mit diesem verbunden zu sein.

Durch diese spezielle Ausführung wird erreicht, dass ein Einfedern des elastischen Elements entlang der Verbindungsachse Z begrenzt und gleichzeitig auch eine Biege- oder Translationsbewegung in der Richtung X eingeschränkt werden kann.

### Vierte Ausführungsform

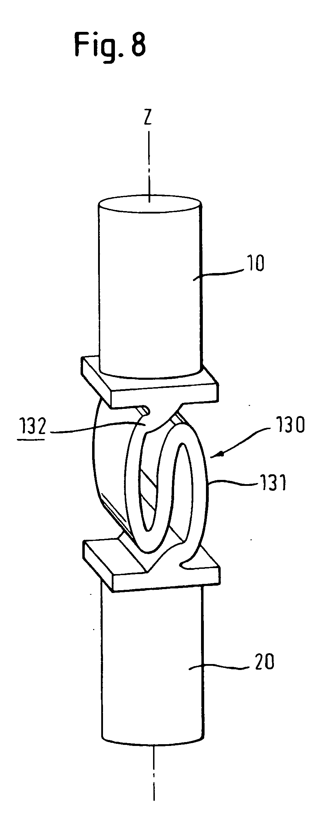

Bei der in Fig. 8 gezeigten vierten Ausführungsform ist im Vergleich zu der in den Fig. 4 und 5 gezeigten zweiten Ausführungsform der mäanderartige Verlauf der Schleifenbäuche 131 des schleifenförmig geformten Flachstabes 132 des elastischen Abschnitts 130 noch stärker ausgeprägt. Durch diese verstärkte Mäanderform weist der Flachstab 132 entlang der Verbindungsachse Z betrachtet in der Mitte des elastischen Abschnitts 130 eine S-förmige Ausbildung auf, sodass in Richtung X zwei Schleifenbäuche 131 nebeneinander liegen.

Bei dieser Ausführungsform ist ein Vorteil, dass der elastische Abschnitt 130 im Vergleich zu den elastischen Abschnitten der ersten bis dritten Ausführungsform verkürzt ist, sodass eine kompaktere Bauweise möglich ist.

### weitere Ausführungsformen und Abwandlungen

Es ist z.B. möglich die Querschnittsformen des elastischen Abschnitts weiter abzuändern oder die Querschnittsform in Verlaufsrichtung des Flachstabes zu verändern. Ebenfalls können das erste und das zweite Ende eine abgewandelte Form aufweisen bzw. müssen nicht zwangsläufig einstückig mit dem elastischen Abschnitt ausgebildet sein.

Ein Einsatz des elastischen Elements in anderen Stabilisierungseinrichtungen für Knochen oder Wirbel als dem in Fig. 9 dargestellten ist selbstverständlich ebenfalls möglich.

Weiterhin sind auch andere Querschnitte des Flachstabes wie z.B. ein rechteckiger Querschnitt mit abgerundeten Kanten möglich.

## Patentansprüche (Patentansprüche für folgende(n) Vertragsstaat(en): DE, ES, FR, GB, IT)

1. Elastisches Element zur Verwendung in einer Stabilisierungseinrichtung für Knochen oder Wirbel mit einem ersten Ende (10) und einem zweiten Ende (20) und einem sich zwischen diesen erstreckenden Abschnitt in Form eines schleifenförmig geformten Stabes (30; 30'; 30"; 130), wobei das erste Ende (10) und das zweite Ende (20) jeweils zylindrisch ausgebildet sind zur Aufnahme in einem Aufnahmeteil (40) eines Kno- chenverankerungselements, **dadurch gekennzeichnet, dass** die Schleifen entlang der Verbindungsachse von dem ersten zu dem zweiten Ende abwechselnd auf zwei entgegengesetzten Seiten der Verbindungsachse (Z) verlaufen wobei der schleifenförmig geformte Stab (30; 30'; 30"; 130) den Querschnitt eines Flachstabes aufweist.

2. Elastisches Element nach Anspruch 1, bei dem der schleifenförmig geformte Stab (30; 30'; 30" ; 130) senkrecht zur Verlaufsrichtung einen im Wesentlichen rechteckigen Querschnitt aufweist.

3. Elastisches Element nach Anspruch 1 oder 2, bei dem zumindest zwei Schleifenbäuche (31a, 31b, 31c; 31'a, 31'b; 31"a, 31"b, 31"c, 31"d; 131) vorgesehen sind, die sich auf gegenüberliegenden Seiten der Verbindungsachse (Z) befinden.

4. Elastisches Element nach einem der Ansprüche 1 bis 3, bei dem der schleifenförmig geformte Stab (30, 30', 30", 130) senkrecht zur Richtung der Schleifenbäuche und senkrecht zur Verbindungsachse (Z) eine gleichmäßige Breite aufweist.

5. Elastisches Element nach einem der Ansprüche 1 bis 4, bei dem der Umfang senkrecht zu Stabachse (Z) im Bereich des ersten Endes (10) oder des zweiten Endes (20) kleiner ist als in dem Abschnitt in Form des schleifenförmig geformten Stabes (30, 30', 30", 130).

6. Elastisches Element nach einem der Ansprüche 1 bis 5, bei dem der Querschnitt des Abschnitts in Form des schleifenförmig geformten Stabes (30, 30', 30", 130) über die gesamte Länge konstant ist.

7. Stabilisierungseinrichtung zur dynamischen Stabilisierung von Knochen, Knochenteilen oder der Wirbelsäule mit zumindest zwei Knochenverankerungselementen, die über ein stabförmiges Element oder eine Platte miteinander verbunden sind, wobei ein Abschnitt oder ein Element der Stabilisierungseinrichtung als ein elastisches Element nach einem der Ansprüche 1 bis 6 ausgebildet ist.

## Patentansprüche (Patentansprüche für folgende(n) Vertragsstaat(en): CH/LI)

1. Elastisches Element zur Verwendung in einer Stabilisierungseinrichtung für Knochen oder Wirbel mit einem ersten Ende (10) und einem zweiten Ende (20) und einem sich zwischen diesen erstreckenden Abschnitt in Form eines schleifenförmig geformten Stabes (30; 30'; 30"; 130), wobei das erste Ende (10) und das zweite Ende (20) jeweils zylindrisch ausgebildet sind zur Aufnahme in einem Aufnahmeteil (40) eines Kno- chenverankerungselements, **dadurch gekennzeichnet, dass** die Schleifen entlang der Verbindungsachse von dem ersten zu dem zweiten Ende abwechselnd auf zwei entgegengesetzten Seiten der Verbindungsachse (Z) verlaufen.

2. Elastisches Element nach Anspruch 1, bei dem der schleifenförmig geformte Stab (30; 30'; 30"; 130) den Querschnitt eines Flachstabes aufweist.

3. Elastisches Element nach einem der Ansprüche 1 oder 2, bei dem der schleifenförmig geformte Stab (30; 30'; 30"; 130) senkrecht zur Verlaufsrichtung einen im Wesentlichen rechteckigen Querschnitt aufweist.

4. Elastisches Element nach einem der Ansprüche 1 bis 3, bei dem zumindest zwei Schleifenbäuche (31a, 31b, 31c; 31'a, 31'b; 31"a, 31"b, 31"c, 31"d; 131) vorgesehen sind, die sich auf gegenüberliegenden Seiten der Verbindungsachse (Z) befinden.

5. Elastisches Element nach einem der Ansprüche 1 bis 4, bei dem der schleifenförmig geformte Stab (30, 30', 30 " , 130) senkrecht zur Richtung der Schleifenbäuche und senkrecht zur Verbindungsachse (Z) eine gleichmäßige Breite aufweist.

6. Elastisches Element nach einem der Ansprüche 1 bis 5, bei dem der Umfang senkrecht zu Stabachse (Z) im Bereich des ersten Endes (10) oder des zweiten Endes (20) kleiner ist als in dem Abschnitt in Form des schleifenförmig geformten Stabes (30, 30', 30" , 130).

7. Elastisches Element nach einem der Ansprüche 1 bis 6, bei dem der Querschnitt des Abschnitts in Form des schleifenförmig geformten Stabes (30, 30', 30", 130) über die gesamte Länge konstant ist.

8. Stabilisierungseinrichtung zur dynamischen Stabilisierung von Knochen, Knochenteilen oder der Wirbelsäule mit zumindest zwei Knochenverankerungselementen, die über ein stabförmiges Element oder eine Platte miteinander verbunden sind, wobei ein Abschnitt oder ein Element der Stabilisierungseinrichtung als ein elastisches Element nach einem der Ansprüche 1 bis 7 ausgebildet ist.

## Claims (Claims for the following Contracting State(s): DE, ES, FR, GB, IT)

1. Elastic element for use in a stabilization device for bones or vertebrae having a first end (10) and a second end (20) and a portion in the form of a loop-shaped formed rod (30; 30'; 30" ; 130) extending between these two ends, wherein the first end (10) and the second end (20) are formed cylindrically, respectively for being received in a receiving part (40) of a bone anchoring element, **characterized in that**, the loops run from the first to the second end along the connection axis, alternating on two opposite sides of the connection axis (Z), wherein the loop-shaped formed rod (30; 30'; 30" ; 130) has the cross-section of a flat rod.

2. Elastic element according to claim 1, wherein the loop-shaped formed rod (30; 30'; 30" ; 130) has a substantially rectangular cross-section perpendicular to the running direction.

3. Elastic element according to claim 1 or 2, wherein at least two loop bellies (31a, 31b, 31c; 31'a, 31'b; 31"a, 31"b, 31"c, 31"d; 131) are provided, which are located on opposite sides of the connection axis (Z).

4. Elastic element according to one of the claims 1 to 3, wherein the loop-shaped formed rod (30; 30'; 30" ; 130) has a continuous width perpendicular to the direction of the loop bellies and perpendicular to the connection axis (Z).

5. Elastic element according to one of the claims 1 to 4, wherein the circumference perpendicular to the rod axis (Z) in the area of the first end (10) or the second end (20) is smaller than in the section in the form of the loop-shaped formed rod (30; 30'; 30" ; 130).

6. Elastic element according to one of the claims 1 to 5, wherein the cross-section of the section in the form of the loop-shaped formed rod (30; 30'; 30" ; 130) is constant over the whole length.

7. Stabilization device for dynamic stabilization of bones, bone parts or the spine having at least two bone anchoring elements, which are connected with each other via a rod-shaped element or a plate, wherein a section or an element of the stabilization device is formed as an elastic element according to one of the claims 1 to 6.

## Claims (Claims for the following Contracting State(s): CH/LI)

1. Elastic element for use in a stabilization device for bones or vertebrae having a first end (10) and a second end (20) and a portion in the form of a loop-shaped formed rod (30; 30'; 30" ; 130) extending between these two ends, wherein the first end (10) and the second end (20) are formed cylindrically, respectively for being received in a receiving part (40) of a bone anchoring element, **characterized in that**, the loops run from the first to the second end along the connection axis, alternating on two opposite sides of the connection axis (Z).

2. Elastic element according to claim 1, wherein the loop-shaped formed rod (30; 30'; 30" ; 130) has the cross-section of a flat rod.

3. Elastic element according to one of the claims 1, or 2, wherein the loop-shaped formed rod (30; 30'; 30" ; 130) has a substantially rectangular cross-section perpendicular to the running direction.

4. Elastic element according to one of the claims 1 to 3, wherein at least two loop bellies (31a, 31b, 31c; 31'a, 31'b; 31"a, 31"b, 31"c, 31"d; 131) are provided, which are located on opposite sides of the connection axis (Z).

5. Elastic element according to one of the claims 1 to 4, wherein the loop-shaped formed rod (30; 30'; 30" ; 130) has a continuous width perpendicular to the direction of the loop bellies and perpendicular to the connection axis (Z).

6. Elastic element according to one of the claims 1 to 5, wherein the circumference perpendicular to the rod axis (Z) in the area of the first end (10) or the second end (20) is smaller than in the section in the form of the loop-shaped formed rod (30; 30'; 30" ; 130).

7. Elastic element according to one of the claims 1 to 6, wherein the cross-section of the section in the form of the loop-shaped formed rod (30; 30'; 30" ; 130) is constant over the whole length.

8. Stabilization device for dynamic stabilization of bones, bone parts or the spine having at least two bone anchoring elements, which are connected with each other via a rod-shaped element or a plate, wherein a section or an element of the stabilization device is formed as an elastic element according to one of the claims 1 to 7.

## Revendications (Revendications pour l'(les) Etat(s) contractant(s) suivant(s): DE, ES, FR, GB, IT)

1. Elément élastique destiné à être utilisé dans un dispositif de stabilisation pour des os ou des vertèbres, avec une première extrémité (10) et une deuxième extrémité (20) et un tronçon s'étendant entre celles-ci et se présentant comme une barre (30 ; 30' ; 30" ; 130) formant des boucles, la première extrémité (10) et la deuxième extrémité (20) étant réalisées chacune avec une forme cylindrique pour être logées dans une pièce de réception (40) d'un élément d'ancrage osseux, **caractérisé en ce que** les boucles sont alternées le long de l'axe de liaison de la première extrémité à la deuxième extrémité, sur deux côtés opposés de l'axe de liaison (Z), la barre (30 ; 30' ; 30" ; 130) formant des boucles présentant la section d'une barre plate.

2. Elément élastique selon la revendication 1, où la barre (30 ; 30' ; 30" ; 130) formant des boucles présente une section transversale sensiblement perpendiculaire à la direction d'extension.

3. Elément élastique selon la revendication 1 ou la revendication 2, où sont prévus au moins deux lobes (31a, 31b, 31c ; 31'a, 31'b ; 31"a, 31"b, 31"c, 31"d; 131) de boucles, disposés sur des côtés opposés de l'axe de liaison (Z).

4. Elément élastique selon l'une des revendications 1 à 3, où la barre (30 ; 30' ; 30" ; 130) formant des boucles présente une largeur constante perpendiculairement à la direction des lobes de boucles et perpendiculairement à l'axe de liaison (Z).

5. Elément élastique selon l'une des revendications 1 à 4, où la circonférence, perpendiculairement à l'axe de barre (Z), est dans la zone de la première extrémité (10) ou de la deuxième extrémité (20), inférieure à celle du tronçon sous forme de barre (30 ; 30' ; 30" ; 130) formant des boucles.

6. Elément élastique selon l'une des revendications 1 à 5, où la section transversale du tronçon sous forme de barre (30 ; 30' ; 30" ; 130) formant des boucles est constante sur toute la longueur.

7. Dispositif de stabilisation pour la stabilisation dynamique d'os, de parties d'os ou de la colonne vertébrale, comportant au moins deux éléments d'ancrage osseux reliés entre eux par un élément en forme de barre ou par une plaque, un tronçon ou un élément du dispositif de stabilisation étant réalisé comme élément élastique selon l'une des revendications 1 à 6.

## Revendications (Revendications pour l'(les) Etat(s) contractant(s) suivant(s): CH/LI)

1. Elément élastique destiné à être utilisé dans un dispositif de stabilisation pour des os ou des vertèbres, avec une première extrémité (10) et une deuxième extrémité (20) et un tronçon s'étendant entre celles-ci et se présentant comme une barre (30 ; 30' ; 30" ; 130) formant des boucles, la première extrémité (10) et la deuxième extrémité (20) étant réalisées chacune avec une forme cylindrique pour être logées dans une pièce de réception (40) d'un élément d'ancrage osseux, **caractérisé en ce que** les boucles sont alternées le long de l'axe de liaison de la première extrémité à la deuxième extrémité, sur deux côtés opposés de l'axe de liaison (Z).

2. Elément élastique selon la revendication 1, où la barre (30 ; 30' ; 30" ; 130) formant des boucles présente la section d'une barre plate.

3. Elément élastique selon la revendication 1 ou la revendication 2, où la barre (30 ; 30' ; 30" ; 130) formant des boucles présente une section transversale sensiblement perpendiculaire à la direction d'extension.

4. Elément élastique selon l'une des revendications 1 à 3, où sont prévus au moins deux lobes (31a, 31b, 31c ; 31'a, 31'b ; 31"a, 31"b, 31"c, 31"d ; 131) de boucles, disposés sur des côtés opposés de l'axe de liaison (Z).

5. Elément élastique selon l'une des revendications 1 à 4, où la barre (30 ; 30' ; 30" ; 130) formant des boucles présente une largeur constante perpendiculairement à la direction des lobes de boucles et perpendiculairement à l'axe de liaison (Z).

6. Elément élastique selon l'une des revendications 1 à 5, où la circonférence, perpendiculairement à l'axe de barre (Z), est dans la zone de la première extrémité (10) ou de la deuxième extrémité (20), inférieure à celle du tronçon sous forme de barre (30 ; 30' ; 30" ; 130) formant des boucles.

7. Elément élastique selon l'une des revendications 1 à 6, où la section transversale du tronçon sous forme de barre (30 ; 30' ; 30" ; 130) formant des boucles est constante sur toute la longueur.

8. Dispositif de stabilisation pour la stabilisation dynamique d'os, de parties d'os ou de la colonne vertébrale, comportant au moins deux éléments d'ancrage osseux reliés entre eux par un élément en forme de barre ou par une plaque, un tronçon ou un élément du dispositif de stabilisation étant réalisé comme élément élastique selon l'une des revendications 1 à 7.
